# EUROPEAN PATENT APPLICATION

(11) **EP 2 014 288 A1**
(43) Date of publication of application: **14.01.2009**
(21) Application number: 07384029.0
(22) Date of filing: 10.07.2007
(51) Int. Cl.: A61K 31/415, A61P 25/04, A61P 11/02, A61K 9/12

(54) **Combination of benzyl-4, 5-dihydro-1H-imidazole derivative and an opioid receptor ligand**

(71) Applicant: Laboratorios del Dr. Esteve S.A., 08041 Barcelona (ES)
(72) Inventor: Romero-Alonso, Luz, 08904 L'Hospitalet de Llobregat(Barcelona) (ES); Pascual-Ramón, Rosalía, 08173 Sant Cugat del Vallés (Barcelona) (ES); Vela-Hernández, José Miguel, 08028 Barcelona (ES); Buschamnn, Dr. Helmut H., 08960 Sant Just Desvern (barcelona) (ES)
(74) Representative: Peters, Hajo

(57) **Abstract**

The present invention refers to a combination of a Compound A, a benzyl-4,5-dihydro-1 H-imidazole derivative according to formula (I) and a Compound B, an opioid receptor ligand; especially of xylometazoline or oxymetazoline and a µ-opioid receptor agonist, most preferably of xylometazoline or oxymetazoline and morphine; a medicament comprising this combination; a pharmaceutical formulation for nasal application comprising this combination; or the use of this combination for the treatment of the symptoms of pain, or the prevention or the prophylaxis of the symptoms of pain, whereas pain particularily encompasses visceral pain, chronic pain, cancer pain, acute pain or neuropathic pain, specifically involving also breakthrough pain.

## Description

### Field of the invention

The present invention refers to a combination of a Compound A, a benzyl-4,5-dihydro-1H-imidazole derivative according to formula (I) and a Compound B, an opioid receptor ligand; especially of xylometazoline or oxymetazoline and a µ-opioid receptor agonist, most preferably of xylometazoline or oxymetazoline and morphine; a medicament comprising this combination; a pharmaceutical formulation for nasal application comprising this combination; or the use of this combination for the treatment of the symptoms of pain, or the prevention or the prophylaxis of the symptoms of pain, whereas pain particularily encompasses visceral pain, chronic pain, cancer pain, acute pain or neuropathic pain, specifically involving also breakthrough pain.

### Background of the invention

The treatment of pain conditions is of great importance in medicine. There is currently a world-wide need for additional pain therapy. The pressing requirement for a specific treatment of pain conditions or as well a treatment of specific pain conditions which is right for the patient, which is to be understood as the successful and satisfactory treatment of pain for the patients, is documented in the large number of scientific works which have recently and over the years appeared in the field of applied analgesics or on basic research on nociception.

PAIN is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210). Even though pain is always subjective its causes or syndromes can be classified.

A long-standing way of treating pain - which is also currently the most common therapeutic approach - is the use of opioids, compounds binding to the receptors of the opioid system (µ, κ, δ and ORL1). These opioids have either been used in clinics or are under investigation with compounds binding to the µ-opioid receptor, like morphine, having currently the highest clinical relevance for the treatment of pain. Nevertheless, even though being highly effective as pain-killers, some of these µ-opioids (as they are also called) do show some severe drawbacks, namely dependency/addiction and tolerance. So, for some of these µ-opioids persons having been exposed for an extended time to compounds like morphine - especially in higher dosages - did show signs of addiction from continuous exposure. On the other hand, in some patients following a prolonged treatment with some of these µ-opioids like morphine the analgesic effect of the treatment severely declined, which is a dramatic situation for these patients given the circumstances of their suffering.

Another aspect that was in the focus of this invention was breakthrough pain (BTP). In patients who have moderate to severe pain, two components are usually present: persistent pain (lasting 12 or more hours/day) and breakthrough pain (BTP), a transitory flare of pain of moderate to severe intensity occurring on a background of otherwise controlled pain. BTP has been reported in up to 86% of patients treated with around-the-clock (ATC) medications for their persistent pain. The onset of BTP is often sudden, reaches maximal intensity within 3 minutes, and lasts for a median duration of 30 minutes. Unfortunately, the medications currently used to treat BTP are often not adequate especially because the onset of pain relief is taking too long, or - in an attempt to to control the BTP in advance - has lead to overdosing of the patients. This has caused considerable research activity in the recent years including efforts being undertaken to develop new ways of applying the active ingredients.

As pain, especially also breakthrough pain, is still considered a major health problem in broad areas of the population and needs a very specific treatment, it was the underlying problem solved by this invention to find new ways of treating pain. Especially it was the underlying problem solved by this invention to improve treatment with µ-opioids especially in regards to the risks of tolerance and addiction and/or allowing also the treatment of breakthrough pain.

So, the main object of this invention is a combination of compounds comprising
a) at least one compound A selected from benzyl-4,5-dihydro-1H-imidazole derivatives according to general formula (I) 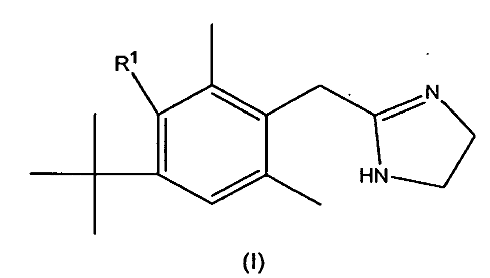 wherein R¹ is selected from hydrogen; or OH;in neutral form, the form of a base or acid, in the form of a salt, preferably a physiologically acceptable salt, in the form of a solvate or of a polymorph;
   and
b) at least one compound B selected from compounds binding to an opoid receptor.

The compound B may be in neutral form, the form of a base or acid, in the form of a salt, preferably a physiologically acceptable salt, in the form of a solvate or of a polymorph and/or in the form of in the form of its racemate, pure stereoisomers, especially enantiomers or diastereomers or in the form of mixtures of stereoisomers, especially enantiomers or diastereomers, in any suitable ratio.

While working on benzyl-4,5-dihydro-1 H-imidazole compounds and their combination with compounds binding to the opioid receptor (especially µ-opioids) it was surprisingly found out that the combination of these compounds either used simultaneously or consecutively could act on the treatment of pain with a high potency, especially showing a super-additive effect in a number of treatments in pain symptoms. Thus this combination was not only strongly ameliorating pain treatment, but seems also to be able to reduce the risk of the development of dependency in a patient especially by allowing a reduction in dose of the opioids and to reduce/deny the effect of development of tolerance.

"Treating" or "treatment" as used in this application are defined as including the treatment of the symptoms of pain, as well as treatment of the disease or disease consequences causing the symptoms, the prevention or the prophylaxis of the symptoms of pain, as well as the prevention or the prophylaxis of the disease or disease consequences causing the symptoms. Preferably "treating" or "treatment" as used in this application are defined as including the treatment of the symptoms of pain, as well as treatment of the disease consequences causing the symptoms, the prevention or the prophylaxis of the symptoms of pain, as well as the prevention or the prophylaxis of the disease consequences causing the symptoms. Most preferably "treating" or "treatment" as used in this application are defined as including the treatment of the symptoms of pain, and the prevention or the prophylaxis of the symptoms of pain.

"Opioids" is the common name for all compounds which have the same mode of action as the constituents of opium, the dried milky liquid of the poppy seed, Papaver somniferum (Brownstein, 1993). All opioids interact in biological systems with the same type of receptor, the so-called opioid receptor. According to the analgesia and side-effect profile four types of opioid receptors, the µ-receptor (ligand = morphine), the κ-receptor (ligand = ketazocine) and the δ-receptor, as well as the later-added ORL1-receptor are known. Corresponding to other receptor systems, binding studies as well as functional investigations indicate that subtypes of opioid receptors exist (Wood, 1982; Pasternak and Wood, 1986). Within the µ-and δ-receptor type 2 subtypes, the µ-1 and µ-2 and δ-1 and δ-2 have been described. The κ-receptor contains an additional κ-3 subtype. By definition all subtypes of the different opioid receptors are included in this invention.

"Compound binding to an opioid receptor" as used in this application is/are defined as having a Kᵢ Value in their binding to at least one of the various opioid-receptor of ≤ 1 µM.

"Compound binding to the µ-opioid receptor" as used in this application is/are defined as having a Kᵢ Value in their binding to the µ-opioid-receptor ≤ 1 µM.

Assays that may be used for determining the affinity and selectivity of a compound binding to the µ-opioid receptor especially a µ-receptor agonist are well known in the art and especially measuring the affinities to these receptors are offered by a number of well-known service companies. A complete comprehensive list of compounds binding to opioid receptors, especially to the µ-opioid receptor, together with their Ki-values for different opioid receptors are given in H.Buschmann et al, Handbook of Pain, (2002), Wiley, and is hereby incorporated by reference and forms part of the disclosure.

The term "solvate" according to this invention is to be understood as meaning any form of a compound in the invention in which this compound has attached to it via non-covalent binding another molecule (most likely a polar solvent) especially including hydrates and alcoholates, e.g. methanolate.

The term "salt" is to be understood as meaning any form of compound A or compound B according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" also means in the context of this invention any salt that is physiologically tolerated (most of the time meaning not being toxic- especially not caused by the counter-ion) if used appropriately for a treatment especially if used on or applied to humans and/or mammals.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid, gluconic acid or citric acid.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

The compound A or compound B of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

The compound A or compound B of the invention may also be in form of a polymorph.

Unless otherwise stated, the compounds in the combination of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

In a peferred embodimdent of the combination according to the invention the compound A is either xylometazoline or oxymetazoline or combination thereof; in neutral form, in the form of a base, or in the form of a salt, preferably a physiologically acceptable salt, or in the form of a solvate or as a polymorph.

Very preferably xylometazoline or oxymetazoline are either in form of the free base or in the form of the hydrochloride.

Xylometazoline is a topical decongestant usually used in the form of xylometazoline hydrochloride in nasal sprays or drops. It is a well-known compound with sympathomimetic properties acting on alpha adrenergic receptors used in nasal sprays for years. Processes for its production are well known in the art. It has the following structure:

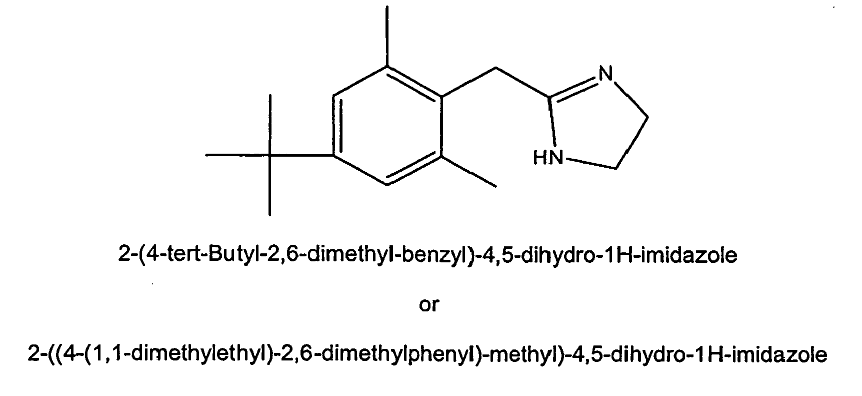

Oxymetazoline is a topical decongestant usually used in the form of oxymetazoline hydrochloride in nasal sprays or drops. It is a well-known compound with sympathomimetic properties acting on alpha adrenergic receptors used in nasal sprays for years. Processes for its production are well known in the art. It has the following structure:

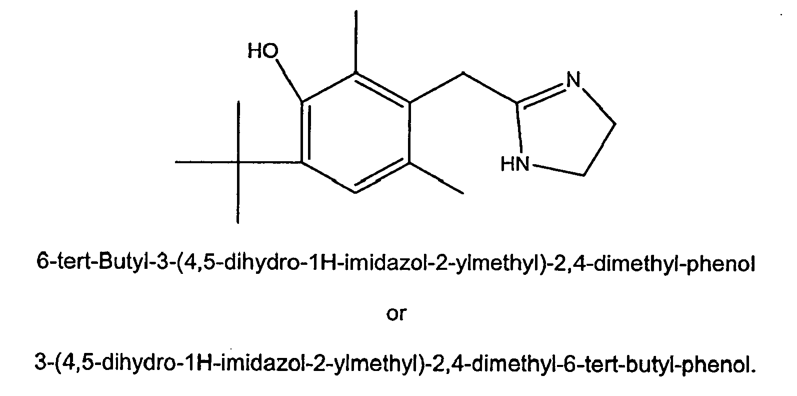

Another especially preferred embodiment of the invention encompasses combinations according to the invention, wherein the compound B is acting as an agonist, preferably a full or a partial agonist or mixed agonist/antagonist on an opioid receptor, preferably on the µ-opioid receptor.

An "Agonist" is defined as a compound that binds to a receptor and has an intrinsic effect, and thus, increases the basal activity of a receptor when it contacts the receptor. Full agonists show the maximum effect on the receptor, whereas a partial agonist is giving less (e.g. 80%) of the response of the full agonist as a maximum. Included in this definition are also compounds acting as mixed agonists/antagonists on the receptor, thus showing agonistic and also antagonistic activity.

A highly preferred embodiment of the invention encompasses combinations according to the invention, wherein
the compound A is either xylometazoline or oxymetazoline or combination thereof; in neutral form, the form of a base or acid, in the form of a salt, preferably a physiologically acceptable salt, in the form of a solvate or of a polymorph
   and
the compound B is acting as an agonist, preferably a full or partial agonist or mixed agonist/antagonist on an opioid receptor, preferably on the µ opioid receptor.

In another preferred embodiment of the combination according to the invention, the compound B binding to an opioid receptor is binding to an opioid receptor, preferably the µ-opioid receptor, with a Ki-value lower than 1000 nM, preferably lower than 200 nM, more preferably lower than 100 nM, even more preferably lower than 50 nM.

In another preferred embodiment of the combination according to the invention, the compound B binding to an opioid receptor is binding to an opioid receptor, preferably to the µ opioid receptor, with a Ki-value lower than 1000 nM, preferably lower than 200 nM, more preferably lower than 100 nM, even more preferably lower than 50 nM, highly preferably lower than 10 nM.

In another preferred embodiment of the combination according to the invention, the compound B binding to the an opioid receptor
is acting as a full or partial agonist on the µ opioid receptor;
   and
is binding to the µ-opioid receptor with a Ki-value lower than 1000 nM, preferably lower than 200 nM, more preferably lower than 100 nM, even more preferably lower than 50 nM.

In another preferred embodiment of the combination according to the invention, the compound B binding to the an opioid receptor
is acting as a full or partial agonist on the µ opioid receptor;
   and
is binding to the µ-opioid receptor with a Ki-value lower than 1000 nM, preferably lower than 200 nM, more preferably lower than 100 nM, even more preferably lower than 50 nM, highly preferably lower than 10 nM.

In another preferred embodiment of the combination according to the invention, the compound B binding to the µ-opioid receptor is selected from
- natural products including semi-synthetic derivatives of natural products, like morphine, codeine and thebain;
- fully synthetic compounds;
- peptides.

In another preferred embodiment of the combination according to the invention, the compound B binding to the µ-opioid receptor is selected from
- morphine, morphine-6-glucoronide, codeine, thebain, papaverin, narcotine,
- heroin, hydromorphone, dihydrocodeine, thebacon, hydrocodone, oxymorphone, oxycodone, ketobemidone, pethidine, anileridine, piminodine, phenoperidine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocine, sufentanil, carfentanil, fentanyl, lofentanil, alfentanil, ohmefentanil, remifentanil, pitramide, benztriamide,diphenoxylate, loperamide, tramadol, tilidine, U-50488, 1-Benzyl-4-(4-bromo-phenyl)-4-dimethylamino-cyclohexanol;
- alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide, remifentanil, sufentanil, tilidine, tramadol, tapentadol,
- Met-enkephalin, Leu-enkephalin, nociceptin, β-endorphin, endomorphin-1, endomorphin-2, metorphamid, dynorphin-A, dynorphin-B, α-neoendorphin.

A highly preferred embodiment of the current invention is a combination according to the invention, wherein the compound A is selected from
xylometazoline, oxymetazoline or combination thereof or their pharmaceutically acceptable salts, solvates or polymorphs;
   and
the compound B is selected from
   - morphine, morphine-6-glucoronide, codeine, thebain, papaverin, narcotine,
   - heroin, hydromorphone, dihydrocodeine, thebacon, hydrocodone, oxymorphone, oxycodone, ketobemidone, pethidine, anileridine, piminodine, phenoperidine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocine, sufentanil, carfentanil, fentanyl, lofentanil, alfentanil, ohmefentanil, remifentanil, pitramide, benztriamide,diphenoxylate, loperamide, tramadol, tilidine, U-50488, 1-Benzyl-4-(4-bromo-phenyl)-4-dimethylamino-cyclohexanol;
   - alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide, remifentanil, sufentanil, tilidine, tramadol, tapentadol,
   - Met-enkephalin, Leu-enkephalin, nociceptin, β-endorphin, endomorphin-1, endomorphin-2, metorphamid, dynorphin-A, dynorphin-B, α-neoendorphin;
preferably in that the compound A is selected from either xylometazoline or oxymetazoline or their physiologically acceptable salts, solvates or polymorphs;
   and
the compound B is selected from buprenorphine, fentanyl, sufentanil, carfentanil, lofentanil, alfentanil, ohmefentanil, remifentanil oxycodone, butorphanol, codeine, morphine-6-glucoronide, or morphine as free base, physiologically acceptable salt or as solvate;
most preferably in that the compound A is either xylometazoline or oxymetazoline or their physiologically acceptable salts, especially the hydrochloride salt, solvates or polymorphs;
   and
the compound B is morphine as free base, physiologically acceptable salt, especially the hydrochloride, gluconate or sulfate salt, or as solvate.

The compounds of group A or B or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

The combination according to the invention is not toxic. Thus a further aspect of the invention refers to a medicament comprising a combination according to the invention, and optionally one or more pharmaceutically acceptable adjuvants.

In human therapeutics, the dose administered can be quite low depending on the route of administration. Generally an effective administered amount of the combination of the invention will depend on the relative efficacy of the combination chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

Any medicament according to the invention contains the active ingredient/combination as well as optionally at least one auxiliary material and/or additive and/or optionally another active ingredient.

The auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

Suitable forms of the pharmaceutical formulation or medicament according to the invention include tablets, sachets, capsules, sprays, gels; solutions or dispersions for injection, inhalation, spraying or ingestion; suppositories, patches, films, lolli-pops, chewing gums etc.

Routes of administration can include intramuscular injection, intraveneous injection, subcutaneous injection, sublingual, intranasal, bucal, patch through skin, oral ingestion, implantable osmotic pump, collagen implants, aerosols or suppository.

Another highly preferred aspect of the invention is relating to a pharmaceutical formulation for nasal application comprising a combination according to the invention, and one or more pharmaceutically acceptable adjuvants.

In the last years big efforts have been undertaken to search for new ways of applying drugs especially analgesics or opioids, aiming in particular at enhancing the onset of action, especially during episodes of "break-through-pain". This is especially described for morphine (Illum et al, JPET, 3001, (1), 391- 400, (2002); Pavis et al., J. of Pain and Symptom Management, 24 (6), 598 - 602 (2002)), for buprenorphine (Lindhardt et al., Int. J. Pharmaceutics, 205, 159-163 (2000) or for butorphanol (Goldstein et al., Headache, 38; 516-522 (1998).

Particularly in this application form the combination of compounds according to the invention do show a further surprisingly high potential knowing that with examples of Compound B like morphine having been tested successfully in clinics (Illum et al, JPET, 3001, (1), 391- 400, (2002); Pavis et al., J. of Pain and Symptom Management, 24 (6), 598 - 602 (2002)) and knowing that the most prominent examples of compound A, xylometazoline or oxymetazoline or their respective hydrochlorides, are used for decades in nasal sprays or drops.

In another embodiment of the pharmaceutical formulation for nasal application according to the invention the pharmaceutical formulation is either in form of a solution, in form of a dispersion, in form of a gas, in form of a gel or in form of a powder, preferably in form of a solution, in form of a dispersion, or in form of a powder.

In another embodiment of the pharmaceutical formulation for nasal application according to the invention the pharmaceutical formulation is comprising at least one pharmaceutically acceptable adjuvants, selected from
- Lubricants, including glycerol, magbesium stearate, macrogolglycerolhydroxystearate;
- Starch- and Cellulose-derivatives, including hypromellose, crystalline cellulose, starch, hydropropoxycellulose;
- Sugar alcohols, including sorbitol;
- Detergents, including salts of EDTA;
- Preservatives, including benzalkonium chloride, citric acid, sodium citrate, or ascorbic acid;
- Solvents, including purified water;
- Buffers, including sodium monohydrogenphosphate/ sodium dihydrogenphosphate,
- Salts, including sodium chloride; and
- Absorption Enhancers, including chitosan, cyclodextrins, or phospholipids.

Also micospheres and nanoparticles may be used in the pharmaceutical formulations-preferably for nasal application - according to the invention.

Other adjuvants for or forms of nasal drug delivery are well-known in the art and are described in e.g.:
- Davis and Illum, Clin. Pharmacokinet. 2003, 42 (13); 1107-1128;
- Bommer, Medical Device Technology, May 1999; 22-28;
- Newman et al., Critical Reviews in Therapeutic Drug Carrier Systems, 2004; 21(1); 21-66;
- Vyas et al., Critical Reviews in Therapeutic Drug Carrier Systems, 2006, 23(4);319-347;
- Illum, J. Pharmaceutical Sciences, 2007; 96(3); 473-484;
- Illum, J. of Controlled Release, 2003; 87; 187-198.

The content of all of these articles are included here by reference.

In another embodiment of the pharmaceutical formulation - preferably for nasal application - according to the invention the pharmaceutical formulation is comprising 30.0 to 0.5 mg/ml of xylometazoline hydrochloride, or 50.0 to 0.5 mg/ml of oxymetazoline hydrochloride, or 30.0 to 0.5 mg/ml of the benzyl-4,5-dihydro-1H-imidazole derivative according to formula (I), preferably xylometazoline or oxymetazoline or their physiologically acceptable salts, especially their hydrochloride salt, solvates or polymorphs.

In another embodiment of the pharmaceutical formulation - preferably for nasal application - according to the invention the pharmaceutical formulation is comprising 400 to 1 mg/ml or preferably 200 to 5 mg/ml of compound B, preferably of buprenorphine, fentanyl, sufentanil, carfentanil, lofentanil, alfentanil, ohmefentanil, remifentanil, oxycodone, butorphanol, morphine-6-glucoronide, or morphine, most preferably of morphine as free base, physiologically acceptable salt, especially the hydrochloride, gluconate or sulfate salt, or as solvate.

In another embodiment of the pharmaceutical formulation - preferably for nasal application - according to the invention the pharmaceutical formulation is comprising a combination according to the invention in a weight per weight ratio of compound A to compound B of 25 to 1 to 0.1 to 1.

In another preferred embodiment of the pharmaceutical formulation - preferably for nasal application - the compound A is selected
- from xylometazoline, oxymetazoline or combination thereof or their pharmaceutically acceptable salts, solvates or polymorphs;
- preferably from either xylometazoline or oxymetazoline or their physiologically acceptable salts, solvates or polymorphs;
- most preferably from either xylometazoline or oxymetazoline or their physiologically acceptable salts, especially the hydrochloride salt, solvates or polymorphs.

In another preferred embodiment of the pharmaceutical formulation - preferably for nasal application - the compound B is selected from
- morphine, morphine-6-glucoronide, codeine, thebain, papaverin, narcotine,
- heroin, hydromorphone, dihydrocodeine, thebacon, hydrocodone, oxymorphone, oxycodone, ketobemidone, pethidine, anileridine, piminodine, phenoperidine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocine, sufentanil, carfentanil, fentanyl, lofentanil, alfentanil, ohmefentanil, remifentanil, pitramide, benztriamide,diphenoxylate, loperamide, tramadol, tilidine, U-50488, 1-Benzyl-4-(4-bromo-phenyl)-4-dimethylamino-cyclohexanol;
- alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide, remifentanil, sufentanil, tilidine, tramadol, tapentadol,
- Met-enkephalin, Leu-enkephalin, nociceptin, β-endorphin, endomorphin-1, endomorphin-2, metorphamid, dynorphin-A, dynorphin-B, α-neoendorphin;
preferably is selected from buprenorphine, fentanyl, sufentanil, carfentanil, lofentanil, alfentanil, ohmefentanil, remifentanil, oxycodone, butorphanol, codeine, morphine-6-glucoronide or morphine as free base, physiologically acceptable salt or as solvate;
most preferably is morphine as free base, physiologically acceptable salt, especially the hydrochloride, gluconate or sulfate salt, or as solvate.

In a further preferred embodiment of the pharmaceutical formulation - preferably for nasal application -
the compound A is selected from xylometazoline, oxymetazoline or combination thereof or their pharmaceutically acceptable salts, solvates or polymorphs;
   and
the compound B is selected from
   - morphine, morphine-6-glucoronide, codeine, thebain, papaverin, narcotine,
   - heroin, hydromorphone, dihydrocodeine, thebacon, hydrocodone, oxymorphone, oxycodone, ketobemidone, pethidine, anileridine, piminodine, phenoperidine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocine, sufentanil, carfentanil, fentanyl, lofentanil, alfentanil, ohmefentanil, remifentanil, pitramide, benztriamide,diphenoxylate, loperamide, tramadol, tilidine, U-50488, 1-Benryl-4-(4-bromo-phenyl)-4-dimethylamino-cyclohexanol;
   - alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide, remifentanil, sufentanil, tilidine, tramadol, tapentadol,
   - Met-enkephalin, Leu-enkephalin, nociceptin, β-endorphin, endomorphin-1, endomorphin-2, metorphamid, dynorphin-A, dynorphin-B, α-neoendorphin;
preferably the compound A is selected from either xylometazoline or oxymetazoline or their physiologically acceptable salts, solvates or polymorphs;
   and
the compound B is selected from buprenorphine, fentanyl, sufentanil, carfentanil, lofentanil, alfentanil, ohmefentanil, remifentanil, oxycodone, butorphanol, codeine, morphine-6-glucoronide or morphine as free base, physiologically acceptable salt or as solvate;
most preferably the compound A is either xylometazoline or oxymetazoline or their physiologically acceptable salts, especially the hydrochloride salt, solvates or polymorphs;
   and
the compound B is morphine as free base, physiologically acceptable salt, especially the hydrochloride, gluconate or sulfate salt, or as solvate.

Another aspect of the invention is a use of at least one combination according to the invention or of xylometazoline or oxymetazoline or their physiologically acceptable salts, solvates or polymorphs for the manufacture of a medicament for the treatment of pain, preferably visceral pain, chronic pain, cancer pain, acute pain, breaktrough pain or neuropathic pain, allodynia or hyperalgesia.

Another aspect of the invention is a use of at least one combination according to the invention for the manufacture of a medicament for the treatment of pain, preferably visceral pain, chronic pain, cancer pain, acute pain, breaktrough pain or neuropathic pain, allodynia or hyperalgesia.

Another aspect of the invention is a use of xylometazoline or oxymetazoline or their physiologically acceptable salts, solvates or polymorphs for the manufacture of a medicament for the treatment of pain, preferably visceral pain, chronic pain, cancer pain, acute pain, breaktrough pain or neuropathic pain, allodynia or hyperalgesia.

In another embodiment of the invention the medicament is used for the treatment of pain in which the stimulus evoking the pain is thermal.

In another embodiment of the invention the medicament is used for the treatment of neuropathic pain - or its subgroups - in which the stimulus evoking the neuropathic pain is thermal.

In another embodiment of the invention the medicament is used for the treatment of pain in which the stimulus evoking the pain is mechanical.

In another embodiment of the invention the medicament is used for the treatment of neuropathic pain - or its subgroups - in which the stimulus evoking the neuropathic pain is mechanical.

Some known subgroups of pain are acute pain, chronic pain, visceral pain and including also headaches, especially migraine. Other subgroups of pain are neuropathic pain, hyperalgesia and allodynia.

"Neuropathic pain" is defined by the IASP as "pain initiated or caused by a primary lesion or dysfunction in the nervous system" (IASP, Classification of chronic pain, 2nd Edition, IASP Press (2002), 210). For the purpose of this invention included under this heading or to be treated as synonymous is "Neurogenic Pain" which is defined by the IASP as "pain initiated or caused by a primary lesion, dysfunction or transitory perturbation in the peripheral or central nervous system". One of the many reasons causing neuropathic pain is diabetes, especially diabetes II.

According to the IASP "allodynia" is defined as "a pain due to a stimulus which does not normally provoke pain" (IASP, Classification of chronic pain, 2nd Edition, IASP Press (2002), 210).

According to the IASP "hyperalgesia" is defined as "an increased response to a stimulus which is normally painful(IASP, Classification of chronic pain, 2nd Edition, IASP Press (2002), 211).

In another preferred embodiment of the use according to the invention the neuropathic pain, is selected from central pain, hyperpathia, peripheral neuropathic pain or peripheral neurogenic pain, causalgia, hyperesthesia, neuralgia, neuritis, or neuropathy.

According to the IASP "central pain" is defined as "a pain initiated or caused by a primary lesion or dysfunction in the central nervous system" (IASP, Classification of chronic pain, 2nd Edition, IASP Press (2002), 211).

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

According to the IASP "hyperesthesia" is defined as "increased sensitivity to stimulation, excluding the senses" (IASP, Classification of chronic pain, 2nd Edition, IASP Press (2002), 211).

According to the IASP "neuralgia" is defined as "Pain in the distribution of a nerve or nerves" (IASP, Classification of chronic pain, 2nd Edition, IASP Press (2002), 212).

According to the IASP "neuritis" is defined as "Inflammation of a nerve or nerves" (IASP, Classification of chronic pain, 2nd Edition, IASP Press (2002), 212).

According to the IASP "neuropathy" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold; Increased response | Stimulus and response rate may be the same or different |

Another aspect of the ionvention refers to the use of at least one combination according to the invention for the manufacture of a medicament for the treatment of sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.

Included in this invention as a very preferred aspect is especially also a method of treatment of a patient or a mammal, including men, suffering from pain by applying to the patient or mammal either a suitable amount of the combination according to the invention comprising at least one compound A and at least one compound B or by applying to the patient or mammal first a suitable amount of compound A followed by applying to the patient or mammal a suitable amount of compound B or by applying to the patient or mammal first a suitable amount of compound B followed by applying to the patient or mammal a suitable amount of compound A.

In a preferred embodiment of this method of treatment according to the invention the patient suffering from pain is suffering from visceral pain, chronic pain, cancer pain, acute pain, breaktrough pain or neuropathic pain, allodynia or hyperalgesia, or more preferably from chronic pain, or cancer pain, and/or from breaktrough pain.

In another preferred embodiment of this method of treatment according to the invention a suitable amount of the combination according to the invention comprising at least one compound A and at least one compound B is applied to the patient in need thereof in form of a pharmaceutical formulation or medicament, optionally followed by either one or more further applications of a suitable amount of the combination according to the invention comprising at least one compound A and at least one compound B, and/or one or more further applications of a suitable amount of compound A and/or compound B.

In another preferred embodiment of this method of treatment according to the invention a suitable amount of compound A is applied to the patient in need thereof, followed - optionally even simultaneously - by application of a suitable amount of compound B, optionally followed by either one or more further applications of a suitable amount of the combination according to the invention comprising at least one compound A and at least one compound B, and/or one or more further applications of a suitable amount of compound A and/or compound B.

In another preferred embodiment of this method of treatment according to the invention a suitable amount of compound B is applied to the patient in need thereof, followed - optionally even simultaneously - by application of a suitable amount of compound A, optionally followed by either one or more further applications of a suitable amount of the combination according to the invention comprising at least one compound A and at least one compound B, and/or one or more further applications of a suitable amount of compound A and/or compound B.

In another preferred embodiment of this method of treatment according to the invention the compound A is selected
- from xylometazoline, oxymetazoline or combination thereof or their pharmaceutically acceptable salts, solvates or polymorphs;
- preferably from either xylometazoline or oxymetazoline or their physiologically acceptable salts, solvates or polymorphs;
- most preferably from either xylometazoline or oxymetazoline or their physiologically acceptable salts, especially the hydrochloride salt, solvates or polymorphs.

In another preferred embodiment of this method of treatment according to the invention the compound B is selected from
- morphine, morphine-6-glucoronide, codeine, thebain, papaverin, narcotine,
- heroin, hydromorphone, dihydrocodeine, thebacon, hydrocodone, oxymorphone, oxycodone, ketobemidone, pethidine, anileridine, piminodine, phenoperidine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocine, sufentanil, carfentanil, fentanyl, lofentanil, alfentanil, ohmefentanil, remifentanil, pitramide, benztriamide,diphenoxylate, loperamide, tramadol, tilidine, U-50488, 1-Benzyl-4-(4-bromo-phenyl)-4-dimethylamino-cyclohexanol;
- alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide, remifentanil, sufentanil, tilidine, tramadol, tapentadol,
- Met-enkephalin, Leu-enkephalin, nociceptin, β-endorphin, endomorphin-1, endomorphin-2, metorphamid, dynorphin-A, dynorphin-B, α-neoendorphin;
preferably is selected from buprenorphine, fentanyl, sufentanil, carfentanil, lofentanil, alfentanil, ohmefentanil, remifentanil, oxycodone, butorphanol, codeine, morphine-6-glucoronide or morphine as free base, physiologically acceptable salt or as solvate;
most preferably is morphine as free base, physiologically acceptable salt, especially the hydrochloride, gluconate or sulfate salt, or as solvate.

In a further preferred embodiment of this method of treatment according to the invention
the compound A is selected from xylometazoline, oxymetazoline or combination thereof or their pharmaceutically acceptable salts, solvates or polymorphs;
   and
the compound B is selected from
   - morphine, morphine-6-glucoronide, codeine, thebain, papaverin, narcotine,
   - heroin, hydromorphone, dihydrocodeine, thebacon, hydrocodone, oxymorphone, oxycodone, ketobemidone, pethidine, anileridine, piminodine, phenoperidine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocine, sufentanil, carfentanil, fentanyl, lofentanil, alfentanil, ohmefentanil, remifentanil, pitramide, benztriamide,diphenoxylate, loperamide, tramadol, tilidine, U-50488, 1-Benzyl-4-(4-bromo-phenyl)-4-dimethylamino-cyclohexanol;
   - alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide, remifentanil, sufentanil, tilidine, tramadol, tapentadol,
   - Met-enkephalin, Leu-enkephalin, nociceptin, β-endorphin, endomorphin-1, endomorphin-2, metorphamid, dynorphin-A, dynorphin-B, α-neoendorphin;
preferably the compound A is selected from either xylometazoline or oxymetazoline or their physiologically acceptable salts, solvates or polymorphs;
   and
the compound B is selected from buprenorphine, fentanyl, sufentanil, carfentanil, lofentanil, alfentanil, ohmefentanil, remifentanil, oxycodone, butorphanol, codeine, morphine-6-glucoronide or morphine as free base, physiologically acceptable salt or as solvate;
most preferably the compound A is either xylometazoline or oxymetazoline or their physiologically acceptable salts, especially the hydrochloride salt, solvates or polymorphs;
   and
the compound B is morphine as free base, physiologically acceptable salt, especially the hydrochloride, gluconate or sulfate salt, or as solvate.

In another preferred embodiment of this method of treatment according to the invention the pharmaceutical formulations or medicaments applied may be prepared to be suitable for intramuscular injection, intraveneous injection, subcutaneous injection oral ingestion, sublingual, intranasal, or bucal application, in form of a patch through skin, an implantable osmotic pump, collagen implants, aerosols or of a suppository. Preferably the pharmaceutical formulations or medicaments applied are suitable for nasal application, sublingual application, oral ingestion or for injection, in highly preferred embodiment suitable for nasal application.

In another preferred embodiment of this method of treatment according to the invention the pharmaceutical formulations or medicaments are in the form of a solution, in form of a dispersion, in form of a gas, in form of a gel or in form of a powder, preferably in form of a solution, in form of a dispersion, or in form of a powder.

In another preferred embodiment of this method of treatment according to the invention the pharmaceutical formulations or medicaments are comprising 30.0 to 0.5 mg/ml of xylometazoline hydrochloride, or 50.0 to 0.5 mg/ml of oxymetazoline hydrochloride or are comprising 30.0 to 0.5 mg/ml of the benzyl-4,5-dihydro-1H-imidazole derivative according to formula (I), preferably xylometazoline or oxymetazoline or their physiologically acceptable salts, especially their hydrochloride salt, solvates or polymorphs.

In another preferred embodiment of this method of treatment according to the invention the pharmaceutical formulations or medicaments are comprising 400 to 1 mg/ml or preferably 200 to 5 mg/ml of compound B, preferably of buprenorphine, fentanyl, sufentanil, carfentanil, lofentanil, alfentanil, ohmefentanil, remifentanil, oxycodone, butorphanol, morphine-6-glucoronide, or morphine, most preferably of morphine as free base, physiologically acceptable salt, especially the hydrochloride, gluconate or sulfate salt, or as solvate.

In another preferred embodiment of this method of treatment according to the invention the pharmaceutical formulations or medicaments are comprising a combination according to the invention in a weith per weight ratio of compound A to compound B of 25 to 1 to 0.1 to 1.

### Pharmacological methods:

### Method 1: Hot-Plate Test

### MATERIAL AND METHODS

### Animals

Male CD1 mice aged from 6 to 8 weeks old are used in these studies. Animals are housed in groups of five, provided with food and water ad libitum and kept in controlled laboratory conditions with the temperature maintained at 21 ± 1 °C and light in 12 h cycles (on at 07:00 h and off at 19:00 h). Experiments are carried out in a soundproof and air-regulated experimental room. The number of mice ranges from 8 to 16 per group in each individual experiment.

### Drugs

Drugs used for treatments are: Morphine and Xylometazoline as well as Oxymetazoline. Doses are calculated based on the molecular weight of the free base or of the respective salts, where applicable.

### EXPERIMENTAL PROCEDURE:

The analgesic activity of the inventive active substance combination is determined in male CDR1 following and adapting the description in the publication of G. Woolfe and A. D. MacDonald, J. Pharm. Exp. Ther. 1944, 80, pages 300-307. The respective description of this publication is incorporated by reference and forms part of the present disclosure.

In accordance to this test the mice are put onto a plate, which is heated to 50 °C and the time is determined until the mice showed signs of pain such as vigorous and repeated licking of the hind or front paws, jumping or pulling back the paws. The mice are kept on the hot plate for no longer than 40 seconds in order to avoid the development of cutaneous lesions. The vehicle, the active substances and the inventive active substance combination to be tested are administered - sometimes in differing concentrations - either by s.c. or by i.p. injection to different groups of mice. 0.5 hours after the administration the animals are put onto the hot plate and the time is measured until they show signs of pain, divided by Front Paw Licking (FPL), Hind Paw Licking (HPL) and Jumping, to allow conclusions on differentiations of pain types through the different pain symptoms/signs of pain. The analgesic efficacy of the active substances or active substance combination is calculated on the basis of the values obtained for the comparison group of mice to which is only administered the vehicle (baseline). By comparing to baseline (defined as 0% analgesia) and to a failure of pain signs (defined as 100% analgesia) Percentage of analgesia is determined. In some cases based on this calculation and with different concentrations of the active substances or active substance combination ED50 values are determined.

### Method 2: Tail Flick Test

The test was performed as previously described (Moncada et al. (2003), Eur. J. Pharmacol. 465, 53-60). Briefly, the animals (male CD1 mice) were restrained in a Plexiglas tube and placed on the tail flick apparatus (LI 7100, Letica, S.A., Spain). A noxious beam of light was focussed on the tail about 4 cm from the tip, and the tail flick latency was recorded automatically to the nearest 0.1 s. The intensity of the radiant heat source was adjusted to yield baseline latencies between 3 and 5 s; this intensity was never changed and any animal whose baseline latency was outside the pre-established limits was excluded from the experiments. In order to minimise injury in the animals, a cut-off time of 10 s was used. The animals received an injection of the vehicle (saline), and drug or drug combination under study, and tail flick latencies were measured at 45 min after drug or vehicle administration. Baseline tail flick latencies were recorded after saline/vehicle injection. By comparing to baseline (defined as 0% analgesia) and to a failure of pain reaction/tail flick (defined as 100% analgesia) Percentage of analgesia was determined.

The examples and figures in the following section describing pharmacological trials are merely illustrative and the invention cannot be considered in any way as being restricted to these applications.

### Figures:

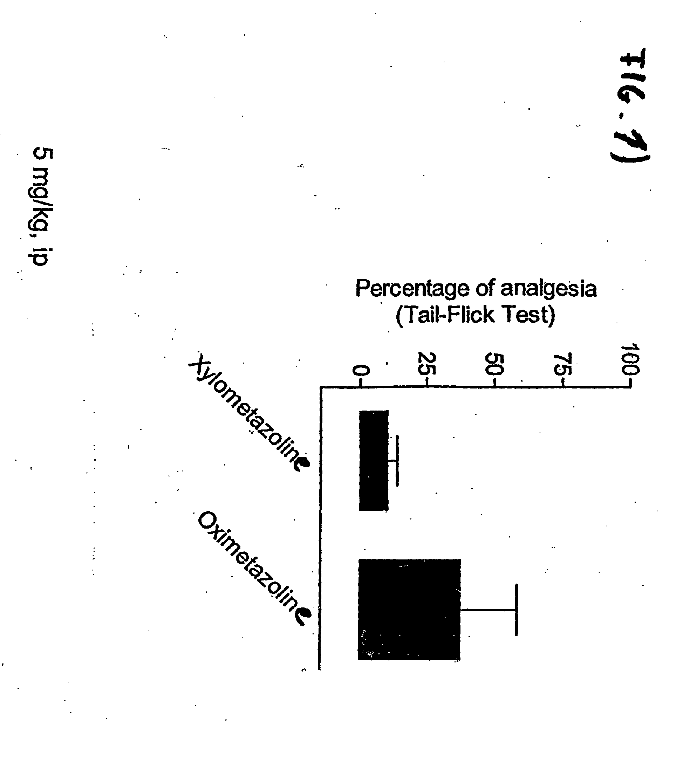

**Figure 1****) Tail-Flick-Test: Xylometazoline and Oxymetazoline**
   Figure 1) refers to Example 1), a Tail-Flick-Test. Male CD1 Mice were receiving i.p. injections of 5mg/kg of the tested substances xylometazoline and oxymetazoline 45 min before measurement. The Baseline was determined based on saline injection.
   Oxymetazoline does show an effect whereas xylometazoline does not show a pronounced activity under this protocoll.
**Figure 2****) Tail-Flick-Test: Morphine in combination with Xylometazoline and Oxymetazoline**

Figure 1) refers to Example 1), a Tail-Flick-Test. Male CD1 Mice were receiving s.c. injections of 2mg/kg morphine and i.p. injections of 5mg/kg of xylometazoline and oxymetazoline 45 min before measurement. The Baseline was determined based on saline injection.

Both combination showed a clearly synergistic effect reaching nearly 100 % analgesia and especially a very pronounced increase over the low-dose morphine applied alone.

### Examples:

### PHARMACOLOGICAL EXAMPLES:

### P-Example 1: Tail-Flick-Test (Combination of Morphine + Xylometazoline and Oxymetazoline):

Male CD1 Mice were receiving:
- s.c. injection of saline (vehicle/baseline);
- s.c. injections of 2 mg/kg morphine;
- i.p.-injection of 5 mg/kg xylometazoline;
- i.p.-injection of 5 mg/kg oxymetazoline;
- s.c. injections of 2 mg/kg morphine + i.p.-injection of 5 mg/kg xylometazoline;
- s.c. injections of 2 mg/kg morphine + i.p.-injection of 5 mg/kg oxymetazoline;

The mice were placed 45 minutes after injection in the tail-flick apparatus. The Baseline was determined based on saline injection. Significance values are determined vs. the saline group and vs. the respective morphine group.

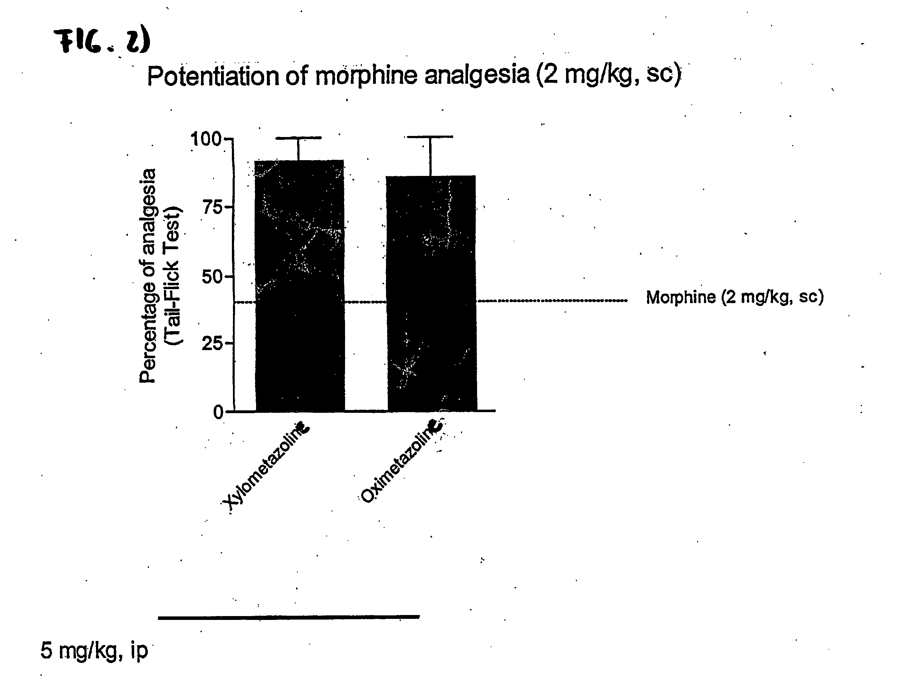

The results are shown in Fig. 1) and 2). The 2 combinations according to the invention did show a highly super-additive effect (see Fig. 2).

### Example 2: Hot-Plate-Test:

Male CD1 Mice are receiving:.
- s.c. injection of saline (vehicle/baseline);
- s.c. injections of 2 mg/kg morphine;
- i.p.-injection of 5 mg/kg xylometazoline;
- i.p.-injection of 5 mg/kg oxymetazoline;
- s.c. injections of 2 mg/kg morphine + i.p.-injection of 5 mg/kg xylometazoline;
- s.c. injections of 2 mg/kg morphine + i.p.-injection of 5 mg/kg oxymetazoline.

The mice are placed 30 minutes after injection on the hot plate heated to 50° C. The signs of pain shown are divided by Front Paw Licking (FPL), Hind Paw Licking (HPL) and Jumping and measured/counted. The Baseline is determined based on saline injection. Significance values are determined vs. the saline group and vs. the respective morphine group.

### Example 3: Hot-Plate-Test:

Male CD1 Mice are receiving
- s.c. injection of saline (vehicle/baseline);
- s.c. injections of 2 mg/kg morphine;
- i.p.-injection of 5 mg/kg xylometazoline;
- i.p.-injection of 5 mg/kg oxymetazoline;
- s.c. injections of 2 mg/kg morphine + i.p.-injection of 5 mg/kg xylometazoline;
- s.c. injections of 2 mg/kg morphine + i.p.-injection of 5 mg/kg oxymetazoline.

The mice are placed 30 minutes after injection on the hot plate heated to 50° C. The signs of pain shown are divided by Front Paw Licking (FPL), Hind Paw Licking (HPL) and Jumping and measured/counted. The Baseline is determined based on saline injection. Significance values are determined vs. the saline group and vs. the respective morphine group.

### Example 4: Hot-Plate-Test after tolerance development:

Male CD1 Mice are devided in 2 groups, the group receiving s.c. injections of morphine in high doses to develop tolerance (hereinafter called "Tolerants"), and the group receiving only vehicle (callede "Non-Tolerants"). The following treatment scheme was followed:

| | Tolerance induction | | | |
|---|---|---|---|---|
| | Day 1 | Day 2 | Day 3 | Day 4 |
| | Morphine (mg/kg sc) | | | |
| Morning | | 30 | 60 | 120 |
| Afernoon | 30 | 45 | 90 | 120 |
| | | | | |
| | | Vehicle | Vehicle | Vehicle |
| Morning Afernoon | Vehicle | Vehicle | Vehicle | Vehicle |

According to past experience after 4 days of treatment the Tolerants group is expressing tolerance towards morphine. Both groups Tolerants and Non-Tolerants are tested on day 5.

On day 5 male CD1 Mice (Tolerants and Non-Tolerants) in two groups each (N=10) are receiving s.c. injections of morphine [2 mg/kg] or s.c. injections of morphine [2 mg/kg] and i.p.-injection of either 5 mg/kg oxymetazoline or 5 mg/kg xylometazoline.

The mice are placed 30 minutes after injection on the hot plate heated to 50° C. The signs of pain shown are divided by Front Paw Licking (FPL), Hind Paw Licking (HPL) and Jumping and measured/counted. The Baseline is determined based on saline injection. Significance values are determined.

### Example 5: Tail-Flick-Test (Combination of Morphine + Chemical Example 1), after tolerance development:

Male CD1 Mice are devided in 2 groups, the group receiving s.c. injections of morphine in high doses to develop tolerance (hereinafter called "Tolerants"), and the group receiving only

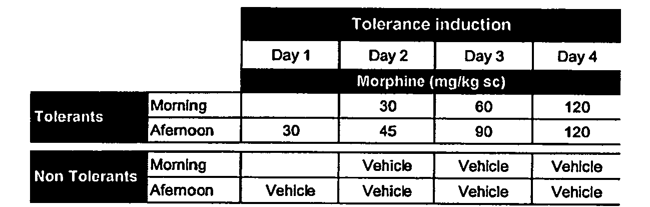

vehicle (called "Non-Tolerants"). The following treatment scheme is followed:

According to past experience after 4 days of treatment the Tolerants group is expressing tolerance towards morphine. Both groups, Tolerants and Non-Tolerants, are tested on day 5.

On day 5 male CD1 Mice (Tolerants and Non-Tolerants) in two groups each (N=10) are receiving s.c. injections of morphine [4 mg/kg] or s.c. injections of morphine [2 mg/kg] and i.p.-injection of either 5 mg/kg oxymetazoline or 5 mg/kg xylometazoline.

The mice are placed 45 minutes after injection in the tail-flick apparatus. The Baseline is determined based on saline injection. Significance values are determined.

### PHARMACEUTICAL FORMULATIONS

### Pharmaceutical Formulation A):

0.25 g of Chitosan glutamate are dissolved in 50 ml of ultrapure water. 2.0 g morphine hydrochloride and 0.185 g of sodium chloride are added together with 1.0 g oxymetazoline hydrochloride and 1 ml of glycerol and the pH is adjusted to pH 4 with 1 M HCl.

### Pharmaceutical Formulation B):

0.25 g of Chitosan glutamate are dissolved in 50 ml of ultrapure water. 2.0 g morphine hydrochloride and 0.185 g of sodium chloride are added together with 0.5 g xylometazoline hydrochloride and the pH is adjusted to pH 4 with 1 M HCl.

### Pharmaceutical Formulation C):

0.25 g of Chitosan glutamate are dissolved in 50 ml of ultrapure water. 2.0 g morphine hydrochloride and 0.185 g of sodium chloride are added together with 2.5 g oxymetazoline hydrochloride and the pH is adjusted to pH 4 with 1 M HCl.

### Pharmaceutical Formulation D):

0.25 g of Chitosan glutamate are dissolved in 50 ml of ultrapure water. 2.0 g morphine hydrochloride and 0.185 g of sodium chloride are added together with 1.5 g xylometazoline hydrochloride and the pH is adjusted to pH 4 with 1 M HCl.

### Pharmaceutical Formulation E):

1.25 g fentanyl citrate are dissolved in 50 ml of ultrapure water and 0.185 g of sodium chloride are added together with 1.0 g oxymetazoline hydrochloride and 1 ml of glycerol and the pH is adjusted to pH 4 with 1 M HCl.

### Pharmaceutical Formulation F):

1.25 g fentanyl citrate are dissolved in 50 ml of ultrapure water and 0.185 g of sodium chloride are added together with 0.5 g xylometazoline hydrochloride and the pH is adjusted to pH 4 with 1M HCl.

### Pharmaceutical Formulation G):

1.25 g fentanyl citrate are dissolved in 50 ml of ultrapure water and 0.185 g of sodium chloride are added together with 2.5 g oxymetazoline hydrochloride and the pH is adjusted to pH 4 with 1M HCl.

### Pharmaceutical Formulation H):

1.25 g fentanyl citrate are dissolved in 50 ml of ultrapure water and 0.185 g of sodium chloride are added together with 1.5 g xylometazoline hydrochloride and the pH is adjusted to pH 4 with 1 M HCl.

## Claims

**1.** A combination of compounds comprising
a) at least one compound A selected from benzyl-4,5-dihydro-1H-imidazole derivatives according to general formula (I) 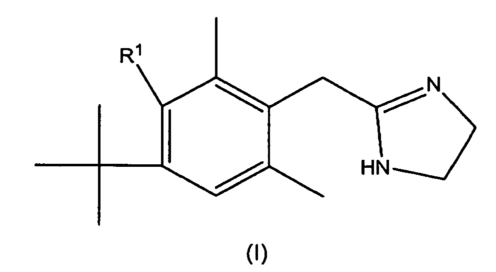 wherein R¹ is selected from hydrogen, or OH;
in neutral form, the form of a base or acid, in the form of a salt, preferably a physiologically acceptable salt, in the form of a solvate or of a polymorph;
and
b) at least one compound B selected from compounds binding to an opoid receptor.

**2.** Combination according to claim 1, in which the compound A is either xylometazoline or oxymetazoline or combination thereof; in neutral form, in the form of a base, or in the form of a salt, preferably a physiologically acceptable salt, or in the form of a solvate or as a polymorph.

**3.** Combination according to claim 1, **characterized in that** the compound B is acting as an agonist, preferably a full or a partial agonist or mixed agonist/antagonist on anopoid receptor, preferably on the µ-opioid receptor.

**4.** Combination according to claim 1, in which
the compound A is either xylometazoline or oxymetazoline or combination thereof; in neutral form, the form of a base or acid, in the form of a salt, preferably a physiologically acceptable salt, in the form of a solvate or of a polymorph
and
the compound B is acting as an agonist, preferably a full or partial agonist or mixed agonist/antagonist on an opioid receptor, preferably on the µ opioid receptor.

**5.** Combination according to any of claims 1, 3 or 4, **characterized in that** the compound B binding to an opioid receptor is binding to an opioid receptor, preferably the µ-opioid receptor, with a Ki-value lower than 1000 nM, preferably lower than 200 nM, more preferably lower than 100 nM, even more preferably lower than 50 nM.

**6.** Combination according to any of claims 1, 3, 4 or 5, **characterized in that** the compound B binding to an opioid receptor
is acting as a full or partial agonist on the µ opioid receptor;
and
is binding to the µ-opioid receptor with a Ki-value lower than 1000 nM, preferably lower than 200 nM, more preferably lower than 100 nM, even more preferably lower than 50 nM.

**7.** Combination according to any of claims 1, or 3 to 6, **characterized in that** the compound B binding to the µ-opioid receptor is selected from
• natural products including semi-synthetic derivatives of natural products, like morphine, codeine and thebain;
• fully synthetic compounds;
• peptides.

**8.** Combination according to any of claims 1, or 3 to 7, **characterized in that** the compound B binding to the µ-opioid receptor is selected from
• morphine, morphine-6-glucoronide, codeine, thebain, papaverin, narcotine,
• heroin, hydromorphone, dihydrocodeine, thebacon, hydrocodone, oxymorphone, oxycodone, ketobemidone, pethidine, anileridine, piminodine, phenoperidine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocine, sufentanil, carfentanil, fentanyl, lofentanil, alfentanil, ohmefentanil, remifentanil, pitramide, benztriamide,diphenoxylate, loperamide, tramadol, tilidine, U-50488, 1-Benzyl-4-(4-bromo-phenyl)-4-dimethylamino-cyclohexanol;
• alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide, remifentanil, sufentanil, tilidine, tramadol, tapentadol,
• Met-enkephalin, Leu-enkephalin, nociceptin, β-endorphin, endomorphin-1, endomorphin-2, metorphamid, dynorphin-A, dynorphin-B, α-neoendorphin.

**12.** Combination according to any of claims 1, 2, 4 or 9 to 11, **characterized in that** the compound A is selected from
xylometazoline, oxymetazoline or combination thereof or their pharmaceutically acceptable salts, solvates or polymorphs;
and
the compound B is selected from
• morphine, morphine-6-glucoronide, codeine, thebain, papaverin, narcotine,
• heroin, hydromorphone, dihydrocodeine, thebacon, hydrocodone, oxymorphone, oxycodone, ketobemidone, pethidine, anileridine, piminodine, phenoperidine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocine, sufentanil, carfentanil, fentanyl, lofentanil, alfentanil, ohmefentanil, remifentanil, pitramide, benztriamide,diphenoxylate, loperamide, tramadol, tilidine, U-50488, 1-Benzyl-4-(4-bromo-phenyl)-4-dimethylamino-cyclohexanol;
• alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide, remifentanil, sufentanil, tilidine, tramadol, tapentadol,
• Met-enkephalin, Leu-enkephalin, nociceptin, β-endorphin, endomorphin-1, endomorphin-2, metorphamid, dynorphin-A, dynorphin-B, α-neoendorphin;
preferably **in that** the compound A is selected from either xylometazoline or oxymetazoline or their physiologically acceptable salts, solvates or polymorphs;
and
the compound B is selected from buprenorphine, fentanyl, sufentanil, carfentanil, lofentanil, alfentanil, ohmefentanil, remifentanil, oxycodone, butorphanol, codeine, morphine-6-glucoronide or morphine as free base, physiologically acceptable salt or as solvate;
most preferably **in that** the compound A is either xylometazoline or oxymetazoline or their physiologically acceptable salts, especially the hydrochloride salt, solvates or polymorphs;
and
the compound B is morphine as free base, physiologically acceptable salt, especially the hydrochloride, gluconate or sulfate salt, or as solvate.

**13.** Medicament comprising a combination according to any of claims 1 to 12, and optionally one or more pharmaceutically acceptable adjuvants.

**14.** Pharmaceutical formulation for nasal application comprising a combination according to any of claims 1 to 12, and one or more pharmaceutically acceptable adjuvants.

**15.** Pharmaceutical formulation for nasal application according to claim 14 being either in form of a solution, in form of a dispersion, in form of a gas, in form of a gel or in form of a powder, preferably in form of a solution, in form of a dispersion, or in form of a powder.

**16.** Pharmaceutical formulation for nasal application according to any of claims 14 to 15 comprising at least one pharmaceutically acceptable adjuvants, selected from
• Lubricants, including glycerol, magbesium stearate, macrogolglycerolhydroxystearate;
• Starch- and Cellulose-derivatives, including hypromellose, crystalline cellulose, starch, hydropropoxycellulose;
• Sugar alcohols, including sorbitol;
• Detergents, including salts of EDTA;
• Preservatives, including benzalkonium chloride, citric acid, sodium citrate, or ascorbic acid;
• Solvents, including purified water;
• Buffers, including sodium monohydrogenphosphate/ sodium dihydrogenphosphate,
• Salts, including sodium chloride; and
• Absorption Enhancers, including chitosan, cyclodextrins, or phospholipids.

**17.** Pharmaceutical formulation according to any of claims 13 to 16 comprising 30.0 to 0.5 mg/ml of xylometazoline hydrochloride, or 50.0 to 0.5 mg/ml of oxymetazoline hydrochloride or 30.0 to 0.5 mg/ml of the benzyl-4,5-dihydro-1H-imidazole derivative according to formula (I), preferably xylometazoline or oxymetazoline or their physiologically acceptable salts, especially their hydrochloride salt, solvates or polymorphs.

**18.** Pharmaceutical formulation according to any of claims 13 to 17 comprising 400 to 1 mg/ml or preferably 200 to 5 mg/ml of compound B, preferably of buprenorphine, fentanyl, sufentanil, carfentanil, lofentanil, alfentanil, ohmefentanil, remifentanil, oxycodone, butorphanol, morphine-6-glucoronide, or morphine, most preferably of morphine as free base, physiologically acceptable salt, especially the hydrochloride, gluconate or sulfate salt, or as solvate.

**19.** Pharmaceutical formulation according to any of claims 13 to 17 comprising a combination according to any of claims 1 to 12 in a weight per weight ratio of compound A to compound B of 25 to 1 to 0.1 to 1.

**20.** Use of at least one combination according to claims 1 to 12, or of xylometazoline or oxymetazoline or their physiologically acceptable salts, solvates or polymorphs for the manufacture of a medicament for the treatment of pain, preferably visceral pain, chronic pain, cancer pain, acute pain, breakthrough pain, or neuropathic pain, allodynia or hyperalgesia.

**21.** Use according to claim 20, wherein the stimulus evoking the pain is mechanical.

**22.** Use according to claim 20, wherein the stimulus evoking the pain is thermal.

**23.** Use, according to any of claims 20 to 22, **characterized in that** the neuropathic pain, is selected from central pain, hyperpathia, peripheral neuropathic pain or peripheral neurogenic pain, causalgia, hyperesthesia, neuralgia, neuritis, or neuropathy.

**24.** Use of at least one combination according to claims 1 to 12, or of xylometazoline or oxymetazoline or their physiologically acceptable salts, solvates or polymorphs for the manufacture of a medicament for the treatment of sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.

## Amended claims

### Amended claims in accordance with Rule 137(2) EPC.

**1.** A combination of compounds comprising
a) at least one compound A selected from benzyl-4,5-dihydro-1H-imidazole derivatives according to general formula (I) 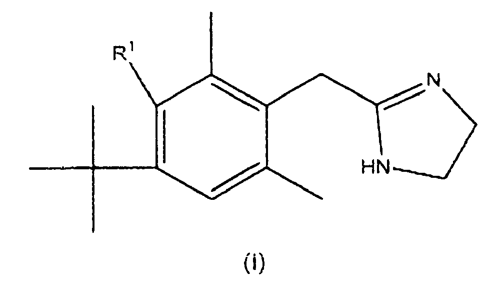 wherein R¹ is selected from hydrogen, or OH;
in neutral form, the form of a base or acid, in the form of a salt, preferably a physiologically acceptable salt, in the form of a solvate or of a polymorph;
and
b) at least one compound B selected from compounds binding to an opoid receptor.

**2.** Combination according to claim 1, in which the compound A is either xylometazoline or oxymetazoline or combination thereof; in neutral form, in the form of a base, or in the form of a salt, preferably a physiologically acceptable salt, or in the form of a solvate or as a polymorph.

**3.** Combination according to claim 1, **characterized in that** the compound B is acting as an agonist, preferably a full or a partial agonist or mixed agonist/antagonist on anopoid receptor, preferably on the µ-opioid receptor.

**4.** Combination according to claim 1, in which
the compound A is either xylometazoline or oxymetazoline or combination thereof; in neutral form, the form of a base or acid, in the form of a salt, preferably a physiologically acceptable salt, in the form of a solvate or of a polymorph
and
the compound B is acting as an agonist, preferably a full or partial agonist or mixed agonist/antagonist on an opioid receptor, preferably on the µ opioid receptor.

**5.** Combination according to any of claims 1, 3 or 4, **characterized in that** the compound B binding to an opioid receptor is binding to an opioid receptor, preferably the µ-opioid receptor, with a Ki-value lower than 1000 nM, preferably lower than 200 nM, more preferably lower than 100 nM, even more preferably lower than 50 nM.

**6.** Combination according to any of claims 1, 3, 4 or 5, **characterized in that** the compound B binding to an opioid receptor
is acting as a full or partial agonist on the µ opioid receptor;
and
is binding to the µ-opioid receptor with a Ki-value lower than 1000 nM, preferably lower than 200 nM, more preferably lower than 100 nM, even more preferably lower than 50 nM.

**7.** Combination according to any of claims 1, or 3 to 6, **characterized in that** the compound B binding to the µ-opioid receptor is selected from
• natural products including semi-synthetic derivatives of natural products, like morphine, codeine and thebain;
• fully synthetic compounds;
• peptides.

**8.** Combination according to any of claims 1, or 3 to 7, **characterized in that** the compound B binding to the µ-opioid receptor is selected from
• morphine, morphine-6-glucoronide, codeine, thebain, papaverin, narcotine.
• heroin, hydromorphone, dihydrocodeine, thebacon, hydrocodone, oxymorphone, oxycodone, ketobemidone, pethidine, anileridine, piminodine, phenoperidine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocinc, sufentanil, carfentanil, fentanyl, lofentanil, alfentanil, ohmefentanil, remifentanil, pitramide, benztriamide, diphenoxylate, loperamide, tramadol, titidine, U-50488, 1-Benzyl-4-(4-bromo-phenyl)-4-dimethylamide-cyclohexanol;
• alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide, remifentanil, sufentanil, tilidine, tramadol, tapentadol.
• Met-enkephalin, Leu-enkephalin, nociceptin, β-endorphin, endomorphin-1, endomorphin-2, metorphamid, dynorphin-A, dynorphin-B, α-neoendorphin.

**9.** Combination according to any of claims 1, or 4, **characterized in that** the compound A is selected from xylometazoline, oxymetazoline or combination thereof or their pharmaceutically acceptable salts, solvates or polymorphs;
and
the compound B is selected from
• morphine, morphine-6-glucoronide, codeine, thebain, papaverin, narcotine,
• heroin, hydromorphone, dihydrocodeine, thebacon, hydrocodone, oxymorphone, oxycodone, ketobemidone, pethidine, anileridine, piminodine, phenopendine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocine, sufentanil, carfentanil, fentanyl, Iofentanil, alfentanil, ohmefentanil, remifentanil, pitramide, benztriamide,diphenoxylate, Ioperamide, tramadol, tilidine. U-50488, 1-Benzyl-4-(4-bromo-phenyl)-4-dimethylamimino-cyclohexanol:
• alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide remifentanil, sufentanil, tilidine, tramadol, tapentadol,
• Met-enkephalin, Leu-enkephalin, nociceptin, β-endorphin, endomorphin-1, endomorphin-2, metorphamid, dynorphin-A, dynorphin-B, α-neoendorphin;
preferably **in that** the compound A is selected from either xylometazoline or oxymetazoline or their physiologically acceptable salts, solvates or polymorphs:
and
the compound B is selected from buprenorphine, fentanyl, sufentanil, carfentanil, lofentanil, alfentanil, ohmefentanil, remifentanil, oxycodone, butorphanol, codeine, morphine-6-glucoronide or morphine as free base, physiologically acceptable salt or as solvate:
most preferably **in that** the compound A is either xylometazoline or oxymetazoline or their physiologically acceptable salts, especially the hydrochloride salt, solvates or polymorphs;
and
the compound B is morphine as free base, physiologically acceptable salt, especially the hydrochloride, gluconate or sulfate salt, or as solvate.

**10.** Medicament comprising a combination according to any of claims 1 to 9, and optionally one or more pharmaceutically acceptable adjuvants.

**11.** Pharmaceutical formulation for nasal application comprising a combination according to any of claims 1 to 9, and one or more pharmaceutically acceptable adjuvants.

**12.** Pharmaceutical formulation for nasal application according to claim 11 being either in form of a solution, in form of a dispersion, in form of a gas, in form of a gel or in form of a powder, preferably in form of a solution, in form of a dispersion, or in form of a powder.

**13.** Pharmaceutical formulation for nasal application according to any of claims 11 to 12 comprising at least one pharmaceutical acceptable adjuvants, selected from
• Lubricants, including glycerol, magbesium stearate, macrogolglycerolhydroxystearate;
• Starch- and Cellulose-derivatives, including hypromellose, crystalline cellulose, starch, hydropropoxycellulose;
• Sugar alcohols, including sorbitol;
• Detergents, including salts of EDTA;
• Preservatives, including benzalkonium chloride, citric acid, sodium citrate or ascorbic acid;
• Solvents, including purified water;
• Buffers, including sodium monohydrogenphosphate/ sodium dihydrogenphosphate,
• Salts, including sodium chloride; and
• Absorption Enhancers, including chitosan, cyclodextrins, or phospholipids.

**14.** Pharmaceutical formulation according to any of claims 10 to 13 comprising 30.0 to 0.5 mg/ml of xylomctazoline hydrochloride, or 50.0 to 0.5 mg/ml of oxymetazoline hydrochloride or 30.0 to 0.5 mg/ml of the benzyl-4,5-dihydro-1H-imidazole derivative according to formula (I), preferably xylometazoline or oxymetazoline or their physiologically acceptable salts, especially their hydrochloride salt, solvates or polymorphs.

**15.** Pharmaceutical formulation according to any of claims 10 to 14 comprising 400 to 1 mg/ml or preferably 200 to 5 mg/ml of compound B, preferably of buprenorphine, fentanyl, sufentanil, carfentanil, lofentanil, alfentanil, ohmefentanil, remifentanil, oxycodone, butorphanol, morphine-6-glucoronide, or morphine, most preferably of morphine as free base, physiologically acceptable salt, especially the hydrochloride, gluconate or sulfate salt, or as solvate.

**16.** Pharmaceutical formulation according to any of claims 10 to 14 comprising a combination according to any of claims 1 to 9 in a weight per weight ratio of compound A to compound B of 25 to 1 to 0.1 to 1.

**17.** Use of at least one combination according to claims 1 to 9, or of xylometazoline or oxymetazoline or their physiologically acceptable salts, solvates or polymorphs for the manufacture of a medicament for the treatment of pain, preferably visceral pain, chronic pain, cancer pain, acute pain, breakthrough pain, or neuropathic pain, allodynia or hyperalgesia.

**18.** Use according to claim 17, wherein the stimulus evoking the pain is mechanical.

**19.** Use according to claim 17, wherein the stimulus evoking the pain is thermal.

**20.** Use, according to any of claims 17 to 19, **characterized in that** the neuropathic pain, is selected from central pain, hyperpathia, peripheral neuropathic pain or peripheral neurogenic pain, causalgia, hyperesthesia, neuralgia, neuritis, or neuropathy.

**21.** Use of at least one combination according to claims 1 to 9, or of xylometazoline or oxymetazoline or their physiologically acceptable salts, solvates or polymorphs for the manufacture of a medicament for the treatment of sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension. irritable bowel syndrome, inflammatory bowel disease, spastic colon or urinary incontinence.
